(19) Europäisches Patentamt
European Patent Office
Office européen des brevets

(11) **EP 1 749 488 B1**

(12) **EUROPÄISCHE PATENTSCHRIFT**

(45) Veröffentlichungstag und Bekanntmachung des Hinweises auf die Patenterteilung:
**19.10.2011 Patentblatt 2011/42**

(21) Anmeldenummer: **06015839.1**

(22) Anmeldetag: **28.07.2006**

(51) Int Cl.:
*A61B 17/225* (2006.01)    *A61B 8/00* (2006.01)
*A61N 7/00* (2006.01)    *G01S 15/00* (2006.01)

(54) **Stosswellentherapiegerät mit Bildgewinnung**

Shockwave therapy apparatus with imaging

Appareil de thérapie par ondes de choc, avec imagerie

(84) Benannte Vertragsstaaten:
**CH DE FR LI**

(30) Priorität: **05.08.2005 DE 102005037043**

(43) Veröffentlichungstag der Anmeldung:
**07.02.2007 Patentblatt 2007/06**

(73) Patentinhaber: **Dornier MedTech Systems GmbH**
**82234 Wessling (DE)**

(72) Erfinder: **Bohris, Christian**
**82152 Krailling (DE)**

(74) Vertreter: **Grünecker, Kinkeldey,**
**Stockmair & Schwanhäusser**
**Leopoldstrasse 4**
**80802 München (DE)**

(56) Entgegenhaltungen:
**WO-A-00/13598      DE-A1- 3 328 039**
**US-A- 5 658 239      US-A- 5 769 790**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Stoßwellentherapiegerät mit einer Stoßwellenquelle zum Aussenden einer Stoßwelle und einer Ultraschalleinheit zur Bildgewinnung mittels Aussendung und Empfang von Ultraschall. Weiterhin betrifft die Erfindung ein Verfahren zum Gewinnen von Bildinformationen für die Stoßwellentherapie.

Bei der Stoßwellentherapie werden beispielsweise Nieren- oder Gallensteine (Konkremente) mittels fokussierter Stoßwellen zertrümmert. Andere Formen der Stoßwellentherapie sind Schmerzbehandlung, Behandlung verschlossener Gefäße, Behandlung des Herzmuskels, etc. in einem entsprechenden Zielgebiet. Die Stoßwellenquelle hat in der Regel einen durch ihre Geometrie vorgegebenen Fokus. Der Fokus kann beispielsweise durch eine Linse erzeugt werden.

Um den Fokus der Stoßwellenquelle auf das Konkrement oder das Zielgebiet zu richten, ist es nötig, das Konkrement oder entsprechende Strukturen des Zielgebiets zu orten. Hierbei ist sowohl die Ortung mittels Röntgenstrahlen als auch mittels Ultraschall bekannt.

Aus der DE 203 15 924 U1 ist beispielsweise eine Stoßwellenquelle bekannt, bei der ein Ultraschallwandler in vorgegebener geometrischer Anordnung mit der Stoßwellenquelle verbunden ist, sodass mit dem Ultraschallwandler das Konkrement geortet werden kann.

Aufgrund der festen geometrischen Beziehung kann die Position des Fokus durch eine Markierung, beispielsweise ein Kreuz, im Ultraschallbild dargestellt werden.

Unter Bildkontrolle lässt sich das Konkrement oder Zielgebiet relativ zum Fokus verschieben, bis es zu einer Übereinstimmung kommt.

Der physikalische Stoßwellenfokus wird allerdings nicht messtechnisch nachgewiesen und dargestellt. Deshalb kann es durch Fehljustage bei der mechanischen Kopplung der Stoßwellenquelle zu Abweichungen zwischen der angezeigten und der tatsächlichen Fokusposition kommen. Dies würde zu uneffektiven Behandlungen mit dem erhöhten Risiko von Nebenwirkungen führen. Außerdem kann nicht unmittelbar nachgewiesen werden, ob die Schockwelle in ausreichendem Maße den Fokus erreicht. Mangelhafte akustische Kopplung zwischen der Schockwelle und dem zu behandelnden Körper oder starke Reflexionen an beispielsweise über dem Zielorgan liegenden Rippen können die applizierte Stoßwellenenergie schwächen.

Aus der DE 37 03 335 C2 ist es bekannt, eine Ultraschallempfangseinheit in Form einer PVDF-Folie vorzusehen, mit der ein reflektiertes Echo empfangen werden kann, das an einem Konkrement beim Auftreffen des Stoßwellenimpulses auf das Konkrement entsteht. Nachteilig ist hierbei, dass eine Bildgewinnung nur bei Applikation eines Stoßwellenimpulses möglich ist, nicht jedoch bereits vorab zur Justage der Stoßwellenquelle. Außerdem setzt dieses Verfahren bestimmte baulich Voraussetzungen an ein Stoßwellentherapiegerät, z.B. das Vorhandensein einer Linse, voraus.

Aus der EP 0 460 536 A1 ist ein Lithotripter bekannt, bei dem zum Überlagern des Fokus und des Konkrements keine Bildgebung eingesetzt wird, sondern mit einer piezoelektrischen Stoßwellenquelle ein schwacher Ultraschallpuls generiert und zeitaufgelöst das Echo aufgenommen wird, sodass aus einem starken Echo darauf geschlossen werden kann, dass die Position des Fokus mit dem Konkrement übereinstimmt.

Nachteilig ist hierbei, dass keine unmittelbare Bildgewinnung möglich ist, die für die Durchführung der Lithotripsie jedoch von großem Vorteil ist, da mit der unmittelbaren Bildgewinnung Form, Größe und Zertrümmerungszustand des Konkrements ermittelt werden können. Darüber hinaus ist die Darstellung der umliegenden Anatomie für eine sichere Behandlung wesentlich, da nur so das Zielgebiet eindeutig identifiziert werden kann, sowie benachbarte Risikostrukturen geschont werden können.

Weiter ist aus der DE 4113697 A1 eine Überlagerung von einem B-Bild mit einem Geschwindigkeitsbild bekannt. Bewegungen von z. B. dem Konkrement oder Kavitationsblasen, die durch die Stoßwelle ausgelöst werden, können so detektiert werden. Nachteilig ist hierbei jedoch, dass Bewegungsvorgänge nur bei an sich ungewünschter Kavitation auftreten oder nur dann wenn das Konkrement von der Stoßwelle auch getroffen wird. Andernfalls sind keine Bewegungen detektierbar und das Verfahren somit nicht weiter hilfreich.

Aus der Druckshift WO 00/13538 ist ein Verfahren gen bestimmung eines Phasenverbeserung von Transducerelementen in hochintensiven fohessierten Ultrashall bekannt.

Das Dokument US 5658 239 offenbart ein verfahren eine Vorrichtung zum Erstellen von Zielkoordimnaten für die Lithotripsy.

Die Schift US 5 769790 betrifft ein System für die fokussierte Ultraschallchirurgie mit Hilfe von Ultraschallbildern.

Aus dem Dokument DE 3328 039 ist eine Zertrümmerungseinrichtung für konkremente im Körper eines Lebewesens bekannt.

Aufgabe der vorliegenden Erfindung ist es, ein Stoßwellentherapiegerät und ein Verfahren zu schaffen, mit denen in möglichst kostengünstiger Weise und möglichst fehlerunanfällig die Position des Fokus und des Konkrements oder des Zielgebiets kontrolliert werden können. Diese Aufgabe wird gelöst, durch ein Stoßwellentherapiegerät nach Anspruch 1 und ein Verfahren nach Anspruch 16.

Bei dem Stoßwellentherapiegerät werden mit ein- und derselben Ultraschalleinheit, die zur normalen Bildgewinnung eingesetzt wird, auch Anteile der Stoßwelle zur Bildinformationsgewinnung registriert, die im Fokusbereich der Stoßwelle reflektiert oder gestreut werden.

Damit ist es möglich, ein Bild des Stoßwellenfokus selbst zu gewinnen. Da dieser Stoßwellenfokus mit der selben Ultraschalleinheit aufgenommen wird, wie das normale Bild ist eine optimale Kontrolle der Behandlung möglich. Der Stoßwellenfokus lässt sich zweifelsfrei mit dem Zielgebiet oder dem Konkrement in Deckung bringen. Durch den Nachweis des Stoßwellenfokus ist sichergestellt, dass die akustische Energie der Therapiewelle den Zielpunkt erreicht.

Die Ultraschalleinheit umfasst bevorzugterweise einen elektrischen Transducer wie etwa einen piezoelektrischen Transducer, der sich elektronisch fokussieren lässt. Dieser ist aus einer Vielzahl von einzelnen Elementen aufgebaut, die entweder 1-dim oder 2-dim array-artig angeordnet sind. Diese Art von Transducem ermöglicht in kostengünstiger Weise eine Ultraschallbildgewinnung mit guter Bildqualität.

Die Ultraschalleinheit kann auch zwei oder mehr piezoelektrische Transducer umfassen, die z. B. verschiedene Frequenzcharakteristiken haben. Jeder Transducer seinerseits kann jedoch aus einer Mehrzahl von Transducerelementen aufgebaut sein. Somit ist es möglich den einen Transducer für eine normale Ultraschallbildgewinnung einzusetzen und den anderen zum Empfang von Anteilen der reflektierten Stoßwelle zur Bildinformationsgewinnung zu verwenden. Die beiden Transducer sind in einer Ultraschalleinheit zusammengefasst, so dass ihre relative Lage zueinander bekannt ist.

Auch kann die Ultraschalleinheit Transducer umfassen, die sich aus Transducerelemengen mit verschiedenen Eigenschaften zusammensetzen. So können beispielsweise zwei oder mehr verschiedenartige Transducerelemente eingesetzt werden, die jeweils verschiedene Frequenzcharakteristiken haben. Die verschiedenen Transducerelemente können dabei beispielsweise immer abwechselnd angeordnet sein. Dies betrifft sowohl 1-dimensionale Anordnungen als auch 2-dimensionale Arrays.

Die verschiedenen erwähnten Ultraschalleinheiten können mit einer elektronischen Signalverarbeitung gekoppelt werden, die sowohl eine Abstrahlfokussierung als auch eine Empfangsfokussierung durchführen kann. Bei der Abstrahlfokussierung werden die einzelnen Elemente der Ultraschalleinheit zeitversetzt aktiviert, sodass sich eine auslaufende Welle ergibt, die fokussiert ist. In gleicher Weise kann bei der Empfangsfokussierung den empfangenen Signalen eine zeitliche Verzögerung elektronisch zugeordnet werden, sodass der Empfang von Ultraschall aus einem bestimmten Bereich (Fokus der Empfangsfokussierung) besonders intensiv ist. Damit ist eine zeilenweise Abtastung sowohl durch Aussendungsfokussierung als auch durch Empfangsfokussierung oder beides zusammen möglich. Für die Darstellung der Stoßwelle kommt die Aussendefokussierung nicht zum Einsatz, da die Stoßwelle nicht von dem Transducer selbst, sondern von der Stoßwellenquelle erzeugt wird. Allerdings lässt sich die Empfangsfokussierung einsetzen, um die von der Stoßwelle erzeugten Echos ortsaufgelöst darzustellen.

Vorteilhafterweise ist das Stoßwellentherapiegerät so ausgeführt, dass die Bildsignalgewinnung mittels Aussendung und Empfang von Ultraschall zu anderen Zeiten erfolgen kann, als die Bildsignalgewinnung durch den Empfang der reflektierten und gestreuten Anteile der Stoßwelle. Dadurch ist eine jeweilige elektronische Signalverarbeitung der verschiedenen Signale möglich.

Für das Bedienpersonal ist es von Vorteil, wenn die verschieden gewonnene Bildinformation überlagert wird, sodass sie in einem einzelnen Bild dargestellt wird. Dadurch ist es insbesondere leicht möglich, die Überlagerung des Stoßwellenfokus mit dem Konkrement zu ermöglichen.

Es sind piezoelektrische Stoßwellenquellen (siehe DE 31 19 295 A1), elektromagnetische (siehe DE 37 03 338 A1) und elektrohydraulische Stoßwellenquellen (siehe DE 36 17 032) bekannt, von denen auch jede bei der vorliegenden Erfindung eingesetzt werden kann.

Die verschiedenen Bauarten können mehr oder weniger starke Ungenauigkeiten in dem Zeitpunkt der Auslösung der Stoßwelle haben. Bei der elektrohydraulischen Stoßwellenquelle hängt die genaue Zeit beispielsweise von dem Abbrandgrad der Elektroden ab.

Vorteilhafterweise ist daher ein entsprechender Sensor vorgesehen, mit dem der genaue Zeitpunkt der Stoßwellenquellenaussendung ermittelt werden kann, sodass die Elektronik der Ultraschalleinheit entsprechend auf den Empfang von reflektierten oder gestreuten Anteilen der Stoßwelle umschalten kann.

Dadurch ist es möglich, einen möglichst lückenlosen Bildgewinnungsfluss zu erreichen, d. h. dass bis unmittelbar vor der Stoßwellenaussendung eine Bildinformationsgewinnung mittels Aussenden und Empfangen des Ultraschalls durch die Ultraschalleinheit erfolgen kann.

Bei Stoßwellenquellen, bei denen der Zeitpunkt der Stoßwelle jedoch genau bekannt ist, da diese durch ein entsprechendes (z.B. elektrisches) Signal ausgelöst wird, kann auch eine unmittelbare Kopplung zwischen der Signalauslösung für die Stoßwellenquelle und dem entsprechenden Betrieb der Ultraschalleinheit für die entsprechende Bildgewinnung durch Anteile der Stoßwelle erfolgen.

Die piezoelektrischen Kristalle des elektronischen Transducers sollen einen möglichst breitbandigen Empfangsbereich besitzen, da dann sowohl der Empfang für das B-Bild als auch der Empfang für die Darstellung der Schockwelle optimiert werden kann. Für die B-Bildgebung sind je nach Tiefe des Bildbereichs Frequenzen zwischen 2 bis 8 MHz geeignet. Die Stoßwelle besitzt häufig ein sehr breites Frequenzspektrum, das von der Art der Stoßwellenerzeugung sowie der Höhe der Stoßwellendruckamplitude abhängig ist. In der Regel ist das Maximum des Frequenzspektrums aber meist unter 1 MHz. Allerdings findet man vor allem im Stoßwellenfokus, wo es aufgrund der hohen Druckamplituden zu nichtlinearen Effekten kommt, auch viele Hochfrequenzanteile im Bereich von 3 bis 5 MHz, die für die Bildgewinnung genutzt werden können. Durch die Verwendung eines breitbandigen Transducers besteht die Möglichkeit, je nach Anwendung die jeweils für den Bildbereich optimale Frequenz auszuwählen. Dies können für die beiden unterschiedlichen Bildinformationen auch unterschiedliche Frequenzen sein. Die Auswahl kann durch akustische oder elektronische Signalfilterung erreicht werden.

Auch können hier Transducer bzw. Transducerelemente mit verschiedenen Frequenzcharakteristiken vorgesehen sein. So können beispielsweise Transducer bzw. Transducerelemente mit einer guten Empfangssensitivität unterhalb von 1 MHz (Empfangssensitivitätsmaximum unterhalb von 1 MHz) vorgesehen sein und andere Transducer bzw. Transducerelemente mit einer guten Empfangssensitivität oberhalb von 1 MHz, wie beispielsweise bei etwa 2, 3, 4, 5, 6, 7, 8, 9 oder 10 MHz. Statt einer guten Empfangssensitivität unter- bzw. oberhalb von 1 MHz kann die gute Empfangssensitivität auch unter- bzw. oberhalb von 0,5, 1,5, 2, 2,5, 3, 3,5, 4, 4,5, 5, 6, 7, 8, 9, 9,5 oder 10 MHz oder dazwischen liegen. Dies hängt von den jeweiligen Anforderungen ab. Die einen Transducer bzw. Transducerelemente werden für die Bildgewinnung mit Stoßwellen optimiert und die anderen für die normale B-Bildgewinnung.

Bei dem Verfahren zum Gewinnen von Bildinformationen wird beispielsweise zur Erstellung eines B-Bildes Ultraschall von einer Ultraschalleinheit ausgesandt und anschließend empfangen. Weiterhin wird mit einer Stoßwellenquelle eine Stoßwelle ausgesandt und die reflektierten oder gestreuten Anteile der Stoßwelle mit der Ultraschalleinheit empfangen, so dass Bildinformation gewonnen werden kann. Vorteilhafterweise kann die Stoßwellenquelle Stoßwellen mit verschiedener Intensität aussenden. So kann beispielsweise zum Justieren eine Stoßwelle mit geringerer Intensität ausgesandt werden, als eine Stoßwelle zum Zertrümmern der Steine. Die Intensität der Stoßwelle zum Justieren muss nur so groß sein, dass die reflektierten und gestreuten Anteile der Stoßwelle mit der Ultraschalleinheit empfangen werden können.

Eine Ausführungsformen des Stoßwellentherapiegeräts und des Verfahrens sollen anhand der Figuren erläutert werden. Dabei zeigt:

Figur 1    eine schematische Darstellung des Stoßwellenfokus,

Figur 2    eine schematische Darstellung der verschiedenen Elemente des Stoßwellentherapiegeräts,

Figur 3    Ortsbezug zwischen Stoßwellenausbreitungsrichtung, Transducer und Bildpunkt bei zentrischer Anordnung der Ultraschalleinheit,

Figur 4    Ortsbezug zwischen Stoßwellenausbreitungsrichtung, Transducer und Bildpunkt, bei einer nicht zentrischen Anordnung der Ultraschalleinheit, wobei die axiale Achse der Stoßwellenquelle und die Ultraschallbildebene in einer Ebene liegen,

Figur 5    eine schematische Darstellung der Überlagerung der verschiedenen Bildinformationen,

Figur 6    eine schematische Darstellung von verschiedenen Ultraschalleinheiten und

Figur 7    eine schematische Darstellung von anderen Ultraschalleinheiten.

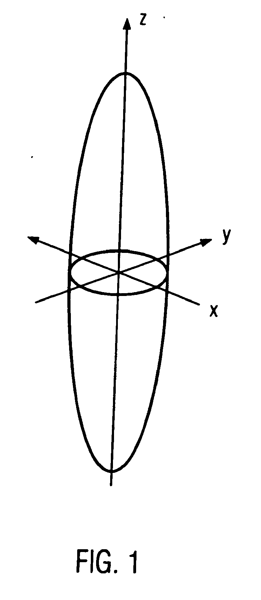

Figur 1 zeigt die ellipsoidförmige Isobare eines Stoßwellenfokus, beispielsweise die auf den Fokusspitzenwert bezogene -6 dB-Isobare, die in Z-Richtung etwa 3 bis 10 cm lang sein kann und in X- und Y-Richtung typischerweise 2 bis 15 mm breit ist.

In der folgenden Beschreibung ist beispielhaft für ein Stoßwellentherapiegerät ein Lithotripter beschrieben. Für die anderen Behandlungsgeräte, wie sie bei der Schmerztherapie, etc. (s. o.) eingesetzt werden, gelten die Ausführungen entsprechend.

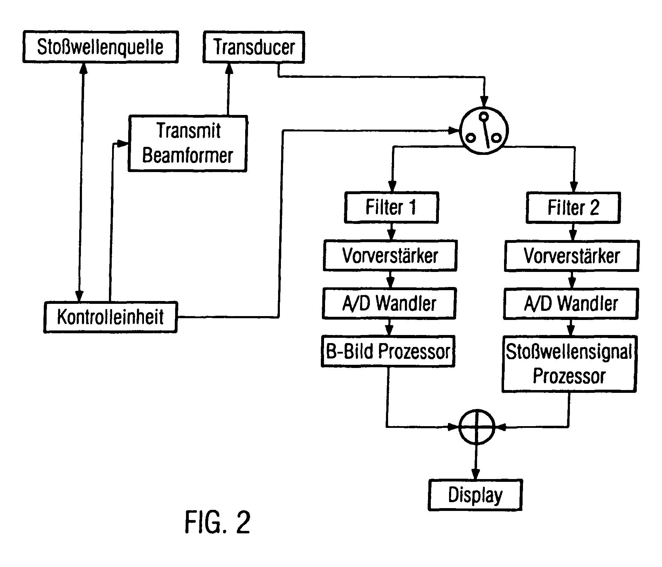

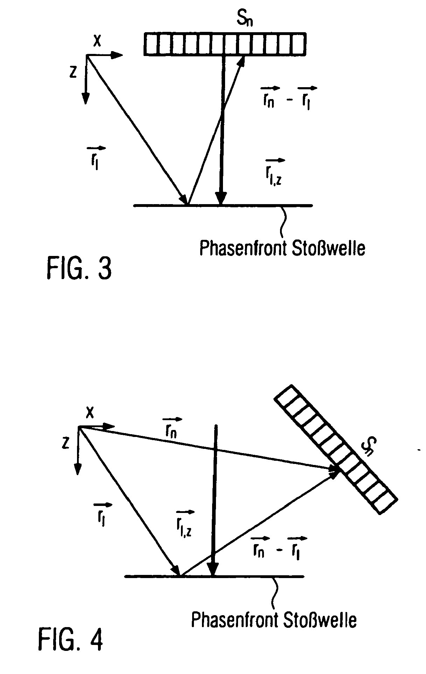

In Figur 2 sind die verschiedenen Elemente des Lithotripters schematisch dargestellt. Der Lithotripter umfasst eine Stoßwellenquelle und einen Transducer, der hier als Ultraschalleinheit dient. Die Ultraschalleinheit kann auf oder neben der Symmetrieachse der Stoßwellenquelle angeordnet sein (s. Figur 3 und 4). Die Ultraschalleinheit kann beispielsweise eine lineare Anordnung von 128 einzelnen piezoelektrischen Elementen sein. Es können auch mehr oder weniger Elemente wie beispielsweise 30, 40, 50, 60, 70, 80, 90, 100, 110, 120, 130, 140, 150, 160, 170, 180, 190 oder 200 oder noch mehr vorgesehen sein.

Die Stoßwellenquelle ist bevorzugt elektromagnetisch.

Eine Kontrolleinheit dient zur Kontrolle der Stoßwellenquelle, sodass zu kontrollierten Zeiten eine Stoßwelle ausgesandt werden kann. Weiterhin kontrolliert die Kontrolleinheit einen Beamformer für das Aussenden von Ultraschall ("Transmit Beamformer"). Dieser steuert den Transducer so an, dass beispielsweise mittels Aussendungsfokussierung Ultraschallpulse zur Gewinnung von Bildinformation auf bestimmten Bildlinien ausgesandt wird. Bei der Aussendefokussierung werden die einzelnen Transducerelemente so zeitlich verzögert angesteuert, dass die einzelnen abgestrahlten Ultraschallwellen - man kann hier auch von Huygens'schen Einzelwellen sprechen - sich im Fokuspunkt konstruktiv überlagern. In der Regel werden die einzelnen Kanäle noch mit einer Apodisierungsfunktion gewichtet, um Nebenmaxima zu unterdrücken. Der ausgesandte Ultraschallpuls wird im Körper gestreut und reflektiert. Die Transducerelemente empfangen diese Reflexe als analoge elektrische Signale. Diese werden einem normalen B-Bild-Prozessor zugeführt. Mit dem normalen B-Bild-Prozessor können übliche Ultraschallbilder erstellt und auf einem Display dargestellt werden.

Wird eine Stoßwelle ausgesandt, kann die Aussendung von Ultraschallpulsen durch den Transducer unterbrochen werden. Aber weiterhin dienen die Transducerelemente dem Empfang. Die Signale, die von den Echos der Stoßwellen resultieren, werden einem Stoßwellen-Signalprozessor zur Bildgewinnung zugeführt. Das kurzzeitige Abschalten der Aussendung von Ultraschallpulsen sowie das wahlweise Zuführen der Signale zu dem B-Bild-Prozessor bzw. Stoßwellensignalprozessor wird durch die Kontrolleinheit gesteuert.

Je nach der weiteren Verarbeitung durch den B-Bild-Prozessor oder durch den Stoßwellensignalprozessor wird das Signal entsprechend gefiltert, verstärkt und digitalisiert. Die getrennte Filterung und Vorverstärkung erlaubt eine jeweils angepasste Signalbehandlung. Ist z. B. die Intensität der beiden Signale deutlich verschieden, so kann dies durch entsprechende unterschiedlich starke Vorverstärkung ausgeglichen werden. Die Filterung kann das Signal auf den jeweils relevanten Frequenzbereich beschränken, um so beispielsweise ein Rauschen zu verringern.

Der Stoßwellensignalprozessor berechnet das Stoßwellenbild. Die Berechnung beruht im Wesentlichen auf einer Empfangsfokussierung, wie sie prinzipiell auch bei der normalen B-Bildgebung zum Einsatz kommt. In Figur 3 ist die Geometrie für den Fall dargestellt, bei dem der Transducer zentrisch zur Stoßwellenquelle sitzt.

Die axiale Achse der Stoßwellenquelle sowie des Transducers sei ohne Beschränkung der Allgemeinheit die Z-Achse. Die Position der Transducerelemente seien $\vec{r}_n$. Um das Signal an einem Bildpunkt $\vec{r}_l$, zu bestimmen, werden die einzelnen Signale $S_n$, die an den Transducerelementen n aufgenommen werden, addiert, wobei die einzelnen Signale relativ zueinander phasenverzögert werden.

Die Phasenverzögerungen sind so zu wählen, dass sie die relativen Wegunterschiede von $\vec{r}_l$, (Ort der Reflexion bzw. Streuung) zu den verschiedenen $\vec{r}_n$ (Empfänger) kompensieren. Die Signalintensität S für den Ort $\vec{r}_l$ lässt sich berechnen nach

$$S(\vec{r}_l) = \sum_{n=1}^{N} Apod_n(\vec{r}_l) \cdot S_n\left(\frac{r_{l,z} + |\vec{r}_n - \vec{r}_l|}{c}\right)$$

(Gleichung 1)

wobei $Apod_n(\vec{r}_l)$ die Apodisierungsfunktion ist, $S_n$ die zeitliche Abhängigkeit der am Ort $\vec{r}_n$ gemessenen Intensität und c die Schallausbreitungsgeschwindigkeit.

Die Apodisierungsfunktion $Apod_n(\vec{r}_l)$ dient zur Unterdrückung von Nebenmaxima und entspricht den Apodisierungen, wie sie in der konventionellen B-Bildgebung eingesetzt werden.

In Gleichung 1 wurde durch die Berücksichtigung von $r_{l,Z}$ angenommen, dass die Stoßwellen eine ebene Phasenfront besitzt. Dies trifft eigentlich nur in der lateralen Ebene des Stoßwellenfokus exakt zu.

Allerdings stellt Gleichung 1 für die besonders interessanten Areale, wie dem Fokusgebiet, sowie auf und nahe der axialen Achse eine hinreichend genaue Näherung dar. Prinzipiell lässt sich auch die Laufzeit der Phasenfront für jeden Punkt $r_l$ angeben und eine exakte Lösung berechnen. Wenn Transducer- und Stoßwellenachse nicht parallel sind, sondern wie in Figur 4 einen Winkel aufweisen, gilt Gleichung 1 ebenso.

Die Berechnung eines Stoßwellenbildes erfordert umfangreiche Rechenarbeit. Da aber die Stoßwellenrate beispielsweise in der Lithotripsie 2 Hz typischerweise nicht übersteigt, lässt sich auch heute schon ein Bild ökonomisch berechnen. Ebenso können, wie bei der konventionellen B-Bildgebung, schnelle Näherungslösungen realisiert werden.

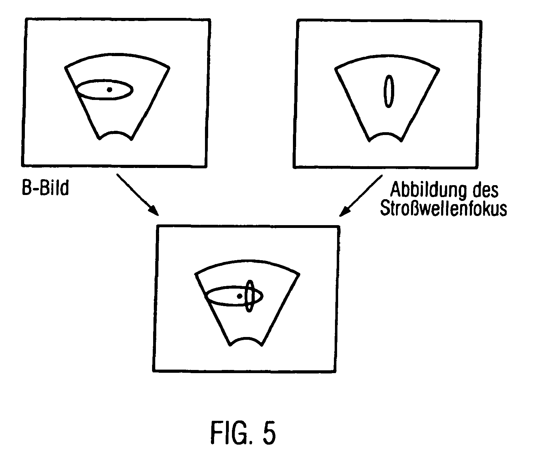

In Figur 5 ist oben links ein normales B-Bild dargestellt, in der ein Organ mit einem Konkrement schematisch dargestellt ist. Das Organ ist ellipsenförmig und das Konkrement darin durch einen schwarzen Punkt dargestellt.

Das Bild des Stoßwellenfokus, wie es mit der voranbeschriebenen Vorrichtung gewonnen werden kann, ist in Figur 5 oben rechts dargestellt. Durch Überlagerung der beiden Bilder, die mit demselben Ultraschalltransducer gewonnen wurden, kann so eine fehlerunanfällige Korrelation zwischen Konkrement und Stoßwellenfokus erreicht werden.

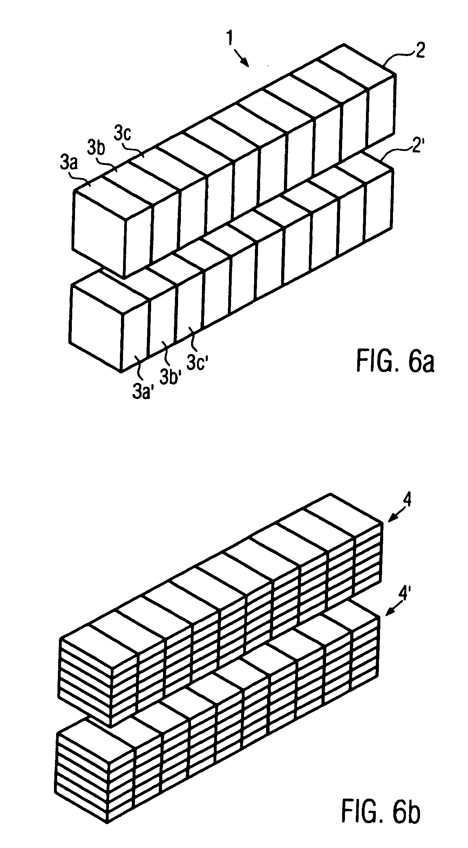

In Figur 6a ist eine Ultraschalleinheit 1 dargestellt, die zwei Transducer 2, 2' umfasst. Jeder Transducer 2, 2' ist aus Transducerelementen 3a, 3b, 3c,..., 3a', 3b', 3c',..... zusammengesetzt. Jedes dieser Transducerelemente 3a, 3b, 3c,..., 3a', 3b', 3c',..... ist beispielsweise ein piezoelektrisches Element. Ggf. haben die Transducerelemente 2 eine andere Größe als die Transducerelemente 2'. Die Transducerelemente des Transducers 2 haben eine andere Frequenzcharakteristik als die Transducerelemente des Transducers 2'. Dadurch kann ein Transducer für die Erzeugung der normalen Ultraschallbilder (etwa B-Bilder) eingesetzt werden, während der andere für den Empfang der reflektierten und/oder gestreuten Stoßwellenanteile eingesetzt wird. Die beiden Transducer können mit ihrer Frequenzcharakteristik (z. B. gegeben durch Resonanzfrequenz und Resonanzbreite) für die jeweiligen Anforderungen getrennt optimiert werden.

Während in Figur 6a der Fall von zwei 1-dimensionalen Transducem 2, 2' gezeigt ist, zeigt die Figur 6b den Fall von zwei 2-dimensionalen array-artigen Transducem 4, 4'.

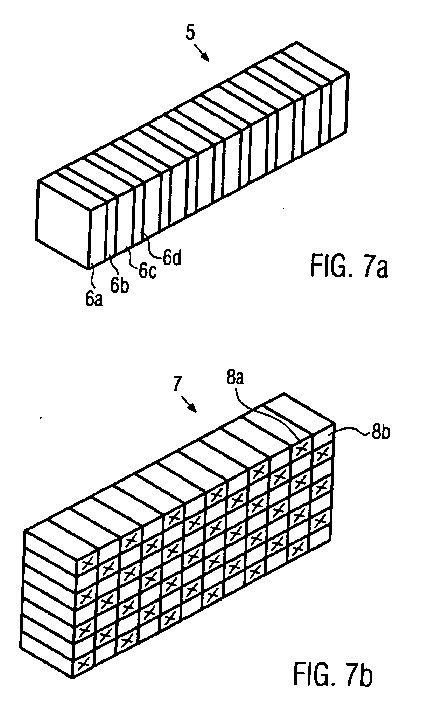

Die Abstrahlrichtung der Transducer in den Figuren 6 und 7 ist beispielsweise jeweils nach rechts. Von rechts kommen auch die einlaufenden akustischen Signale, die empfangen werden sollen.

In Figur 7a ist ein Transducer 5 mit zwei verschiedenartigen Transducerelementen 6a, 6b, 6c, 6d gezeigt. Die Transducerelemente 6a und 6c sind kleiner als die Transducerelemente 6b und 6d. Die verschiedenenartigen Transducerelemente müssen jedoch nicht unbedingt eine verschiedene Größe haben, sie können sich z.B. stattdessen oder zusätzlich auch durch ihre Frequenzcharakteristik unterscheiden. Die einen Elemente 6a, 6c können zum Erzeugen der normalen Ultraschallbilder (etwa B-Bilder) eingesetzt werden, während die anderen 6b, 6d für den Empfang der reflektierten und/oder gestreuten Stoßwellenanteile eingesetzt werden.

In Figur 7b ist der Fall eines 2-dimensionalen Tranducers 7 gezeigt, bei dem verschiedenartige Transducerelemente 8a, 8b nebeneinander angeordnet sind, hier speziell in Form eines Schachbrettmusters. Die einen Transducerelemente 8a sind zur Kennzeichnung mit einem Kreuz versehen, während die anderen 8b ohne Kennzeichen sind.

Bei dem Verfahren wird mit dem Transducer in Figur 2 Ultraschall ausgesandt und empfangen. Der empfangene Ultraschall wird mit dem Transducer in ein elektrisches Signal umgewandelt. Eine Kontrolleinheit steuert einen Schalter so, dass das Signal nach Filterung, Vorverstärkung und Digitalisierung einem B-Bild-Prozessor zugeführt wird. Dieser wertet die Bilder in herkömmlicher Weise aus und führt sie einem Display zu, sodass mit dem Transducer ein Abbild eines Konkrements erhalten wird.

Zur Aussendungsfokussierung wird hierbei ein Transmit Beamformer eingesetzt, der von einer Kontrolleinheit gesteuert wird.

Die Kontrolleinheit löst anschließend die Aussendung einer Stoßwelle von der Stoßwellenquelle aus. Die Aussendung von Ultraschall mit dem Transmit Beamformer und dem Transducer wird dabei unterbrochen. Weiterhin führt die Kontrolleinheit die ab diesem Zeitpunkt empfangenen Signale des Transducers dem Stoßwellensignalprozessor zu. Die ausgesandte Stoßwelle wird in dem Medium, in das sie ausgesandt wurde, reflektiert und gestreut. Diese reflektierten und/oder gestreuten Anteile werden von dem Transducer empfangen, in elektrische Signale umgewandelt und durch den Filter 2, einen Vorverstärker und einen A/D-Wandler dem Stoßwellensignalprozessor zugeführt. Sobald diese Signale abgeklungen sind oder nach einer voreingestellten Zeit, wird durch die Kontrolleinheit der Transmit Beamformer und der Transducer wieder zu normalen Bildgewinnung (B-Bild) angesteuert und die Signale des A/D-Wandlers werden dem B-Bild-Prozessor zugeführt, sodass weiter kontinuierlich Ultraschallbilder gewonnen werden.

Falls für die B-Bildgewinnung und die Signalgewinnung für den Stoßwellensignalprozessor verschiedene Transducer oder Transducerelemente eingesetzt werden, kann der Schalter, der von der Kontrolleinheit gesteuert wird, entfallen. Beide Arten der Bildgewinnung können dann gleichzeitig nebeneinander durchgeführt werden. Das bedeutet auch, dass die B-Bildgewinnung während der Stoßwellenemission nicht unterbrochen werden muss. Falls verschiedene Transducerelemente eines Transducers eingesetzt werden, sollte jedoch eine geeignete Aufteilung der Signale auf den B-Bild-prozessor und den Stoßwellensignalprozessor vorgenommen werden, und zwar entweder der digitalisierten Daten oder der analogen Signale. Auch eine entsprechend getrennte Verkabelung der verschiedenartigen Transducerelemente kann hier vorgesehen sein.

Durch Überlagerung der Bildinformation, die mit dem B-Bild-Prozessor und Stoßwellensignalprozessor (siehe Figur 2) gewonnen wurde (was beispielsweise durch einfache Addition von Bildsignalen geschehen kann), können diese, wie in Figur 5 dargestellt, auf einem einzelnen Bildschirm in Überlagerung dargestellt werden. Die verschiedenen Bildinformationen können vorteilhafterweise in ihrer Helligkeit und sonstigen Bildparametem getrennt eingestellt werden, damit keine der Bildinformationen die andere völlig überstrahlt. Auch eine Darstellung in verschiedenen Farben ist möglich. Durch die Überlagerung der Bildinformationen ist unmittelbar die Lage des Stoßwellenfokus und des Konkrements ersichtlich, so dass eine Ein- bzw. Nachstellung der Stoßwellenquelle bzw. des Stoßwellenfokus erfolgen kann.

Die Bildgewinnung durch Aussenden und Empfang von Ultraschall sowie durch Empfang von reflektierten oder gestreuten Anteilen der Stoßwelle kann mehrmals hintereinander durchgeführt werden. Typischerweise werden bei einer Lithotripsiebehandlung 1000 bis 2000 Stoßwellen ausgesandt. Für jede Stoßwelle wird einmal zwischen den verschiedenen Arten der Bildgewinnung hin- und wieder zurückgeschaltet, falls nicht beide Bildgewinnungen gleichzeitig betrieben werden.

Die Ultraschalleinheit kann im Prinzip im Verhältnis zu der Stoßwellenquelle beliebig angeordnet sein. Vorteilhaft ist jedoch eine Anordnung derart, dass der Ultraschall den selben Weg nimmt wie die Stoßwellenquelle, um so das Medium, das die Stoßwelle durchläuft, selbst mit dem Ultraschall darstellen zu können. Dies kann beispielsweise dadurch erreicht werden, dass die Ultraschalleinheit zentrisch vor der Stoßwellenquelle angeordnet ist.

Dies ist beispielsweise für die Erkennung von Kavitationsbläschen etc., die einen negativen Einfluss auf die Stoßwelle haben, vorteilhaft.

**Patentansprüche**

1. Stoßwellentherapiegerät mit:

   einer Stoßwellenquelle zum Aussenden einer Stoßwelle und
   einer Ultraschalleinheit (1) zur Bildinformationsgewinnung mittels Aussendung und Empfang von Ultraschall, wobei
   die Ultraschalleinheit zum Empfang von reflektierten und/oder gestreuten Anteilen der Stoßwelle für die Bildinformationsgewinnung vorgesehen ist,
   **dadurch gekennzeichnet, dass**
   mit der Ultraschalleinheit eine Bildinformationsgewinnung eines Stoßwellenfokus möglich ist.

2. Stoßwellentherapiegerät nach Anspruch 1, **dadurch gekennzeichnet, dass** die Ultraschalleinheit einen oder mehrere Transducer (2; 2'; 4; 4'; 5; 7) umfasst, der/die seiner/ihrerseits jeweils mehrere Transducerelemente (3a, 3b; 3c; 3a'; 6a; 6b; 8a; 8b), wie etwa piezoelektrische Elemente, umfasst/umfassen.

3. Stoßwellentherapiegerät nach Anspruch 2, **dadurch gekennzeichnet, dass** die elektrischen Transducerelemente (3a, 3b; 3c; 3a'; 6a; 6b; 8a; 8b) auf einer geraden und/oder gekrümmten wie etwa kreisbogenförmigen Linie angeordnet sind und/oder auf einer ebenen und/oder gekrümmten Fläche angeordnet sind.

4. Stoßwellentherapiegerät nach einem der Ansprüche 1 bis 3, **dadurch gekennzeichnet, dass** die Ultraschalleinheit Transducer (2; 2'; 4; 4'; 5; 7) mit verschiedenen Frequenzcharakteristika umfasst und/oder dass ein oder mehrere Transducer Transducerelemente (3a, 3b; 3c; 3a'; 6a; 6b; 8a; 8b) mit verschiedenen Frequenzcharakteristika umfassen.

5. Stoßwellentherapiegerät nach einem der Ansprüche 1 bis 4, **dadurch gekennzeichnet, dass** eine elektronische Signalverarbeitung der von der Ultraschalleinheit empfangenen Signale vorgesehen ist, mit der eine Empfangsfokussierung durchgeführt werden kann.

6. Stoßwellentherapiegerät nach einem der Ansprüche 1 bis 5, **dadurch gekennzeichnet, dass** die Aussendung und der Empfang des Ultraschalls zu anderen Zeiten und/oder gleichzeitig erfolgen kann, wie der Empfang der reflektierten und/oder gestreuten Anteile einer Stoßwelle.

7. Stoßwellentherapiegerät nach einem der Ansprüche 1 bis 6, **dadurch gekennzeichnet, dass** die durch Aussendung und Empfang gewonnene Bildinformation sowie die durch den Empfang von reflektierten und/oder gestreuten Anteilen der Stoßwelle gewonnenen Bildinformation zur Darstellung in einem einzelnen Bild verarbeitet wird.

8. Stoßwellentherapiegerät nach einem der Ansprüche 1 bis 7, **dadurch gekennzeichnet, dass** die Bildinformationsgewinnung mittels Aussendung und Empfang von Ultraschall zur Erzeugung eines B-Bildes dient.

9. Stoßwellentherapiegerät nach einem der Ansprüche 1 bis 8, **dadurch gekennzeichnet, dass** ein Sensor zur Erfassung des Zeitpunkts des Aussendens einer Stoßwelle vorgesehen ist.

10. Stoßwellentherapiegerät nach einem der Ansprüche 1 bis 9, **dadurch gekennzeichnet, dass** die Ultraschalleinheit zum Empfang von Ultraschall mit einer Frequenz zwischen 1 MHz und 8 MHz vorgesehen ist.

11. Stoßwellentherapiegerät nach einem der Ansprüche 1 bis 10, **dadurch gekennzeichnet, dass** erste Transducerelemente (3a; 3b; 3c; 6a; 6c; 8a) vorgesehen sind, die ein Empfangssensitivitätsmaximum unterhalb von einer spezifischen Frequenz aufweisen und dass zweite Transducerelemente (3a'; 3b'; 3c'; 6b; 6d; 8b) vorgesehen sind, die ein Empfangssensitivitätsmaximum oberhalb von der spezifischen Frequenz aufweisen, wobei die spezifische Frequenz beispielsweise bei 0,5, 1, 1,5, 2, 2,5, 3, 3,5, 4, 4,5, 5, 5,5, 6, 6,5, 7, 7,5 8, 8,5, 9, 9,5 oder 10 MHz oder dazwischen liegt.

**12.** Stoßwellentherapiegerät nach einem der Ansprüche 1 bis 11, **dadurch gekennzeichnet, dass** die Stoßwellenquelle Stoßwellen mit einem Intensitätsmaximum bei einer Frequenz unterhalb von 1 MHz ausgibt.

**13.** Stoßwellentherapiegerät nach einem der Ansprüche 1 bis 12, **dadurch gekennzeichnet, dass** die Stoßwellenquelle eine elektromagnetische Stoßwellenquelle ist.

**14.** Stoßwellentherapiegerät nach einem der Ansprüche 1 bis 13, **dadurch gekennzeichnet, dass** die Ultraschalleinheit zentrisch vor der Stoßwellenquelle angeordnet ist.

**15.** Verfahren zum Gewinnen von Bildinformationen für die Stoßwellentherapie mit den Schritten:

a) Aussenden von Ultraschall mit einer Ultraschalleinheit (1)
b) Empfangen der Reflexionen des ausgesandten Ultraschalls mit der Ultraschalleinheit zur Bildinformationsgewinnung
c) Aussenden einer Stoßwelle mit einer Stoßwellenquelle
**gekennzeichnet durch**
d) Empfangen von reflektierten und/oder gestreuten Anteilen der Stoßwelle mit der Ultraschalleinheit zur Bildinformationsgewinnung des Stoßwellenfokuses.

**16.** Verfahren nach Anspruch 15, **dadurch gekennzeichnet, dass** derselbe Transducer (2; 2'; 4; 4'; 5; 7) der Ultraschalleinheit die reflektierten und/oder gestreuten Anteile der Stoßwelle zur Weiterverarbeitung der Signale empfängt, der auch die Reflexionen des ausgesandten Ultraschalls zur Weiterverarbeitung empfängt.

**17.** Verfahren nach einem der Ansprüche 15 oder 16, **dadurch gekennzeichnet, dass** ein Transducer (2; 4) der Ultraschalleinheit die reflektierten und/oder gestreuten Anteile der Stoßwelle zur Weiterverarbeitung empfängt und ein anderer Transducer (2'; 4') der Ultraschalleinheit die Reflexionen des ausgesandten Ultraschalls zur Weiterverarbeitung empfängt.

**18.** Verfahren nach einem der Ansprüche 15 bis 17, **dadurch gekennzeichnet, dass** die Aussendung und der Empfang des Ultraschalls zu anderen Zeiten und/oder gleichzeitig erfolgt, wie der Empfang der reflektierten und/oder gestreuten Anteile einer Stoßwelle.

**Claims**

**1.** Shockwave therapy device with:

a shockwave source for emitting a shockwave and
an ultrasonic unit (1) for obtaining image information by means of emission and reception of ultrasound, wherein the ultrasonic unit is provided for the reception of reflected and/or scattered parts of the shockwave for obtaining image information,
**characterised in that** obtaining image information of a shockwave focus is possible with the ultrasonic unit.

**2.** Shockwave therapy device according to claim 1, **characterised in that** the ultrasonic unit comprises one or more transducers (2; 2'; 4; 4'; 5; 7) which for its/their part each comprises/comprise a plurality of transducer elements (3a, 3b; 3c; 3a'; 6a; 6b; 8a; 8b), such as for example piezoelectric elements.

**3.** Shockwave therapy device according to claim 2, **characterised in that** the electrical transducer elements (3a, 3b; 3c; 3a'; 6a; 6b; 8a; 8b) are arranged on a straight and/or curved, such as for example arc-shaped, line and/or are arranged on a flat and/or curved surface.

**4.** Shockwave therapy device according to one of claims 1 to 3, **characterised in that** the ultrasonic unit comprises transducers (2; 2'; 4; 4'; 5; 7) with different frequency characteristics and/or that one or more transducers comprise transducer elements (3a, 3b; 3c; 3a'; 6a; 6b; 8a; 8b) with different frequency characteristics.

**5.** Shockwave therapy device according to one of claims 1 to 4, **characterised in that** an electronic signal processing of the signals received by the ultrasonic unit is provided with which a reception focussing can be carried out.

6. Shockwave therapy device according to one of claims 1 to 5, **characterised in that** the emission and reception of the ultrasound can take place at different times and/or at the same time as the reception of the reflected and/or scattered parts of a shockwave.

7. Shockwave therapy device according to one of claims 1 to 6, **characterised in that** the image information produced by emission and reception and the image information produced by the reception of reflected and/or scattered parts of the shockwave is processed for representation in a single image.

8. Shockwave therapy device according to one of claims 1 to 7, **characterised in that** the production of image information by means of emission and reception of ultrasound is used to create a B-scan.

9. Shockwave therapy device according to one of claims 1 to 8, **characterised in that** a sensor is provided to record the time of emission of a shockwave.

10. Shockwave therapy device according to one of claims 1 to 9, **characterised in that** the ultrasonic unit for the reception of ultrasound is provided with a frequency between 1 MHz and 8 MHz.

11. Shockwave therapy device according to one of claims 1 to 10, **characterised in that** first transducer elements (3a, 3b; 3c; 6a; 6c; 8a) that have a reception sensitivity maximum below a specific frequency are provided and that second transducer elements (3a'; 3b'; 3c'; 6b; 6d; 8b) that have a reception sensitivity maximum above the specific frequency are provided, wherein the specific frequency is for example 0.5, 1, 1.5, 2, 2.5, 3, 3.5, 4, 4.5, 5, 5.5, 6, 6.5, 7, 7.5, 8, 8.5, 9, 9.5 or 10 MHz or in between.

12. Shockwave therapy device according to one of claims 1 to 11, **characterised in that** the shockwave source emits shockwaves with an intensity maximum at a frequency below 1 MHz.

13. Shockwave therapy device according to one of claims 1 to 12, **characterised in that** the shockwave source is an electromagnetic shockwave source.

14. Shockwave therapy device according to one of claims 1 to 13, **characterised in that** the ultrasonic unit is arranged centrically in front of the shockwave source.

15. Method for producing image information for the shockwave therapy with the steps:

   a) emitting ultrasound with an ultrasonic unit (1)
   b) receiving reflections of the emitted ultrasound with the ultrasonic unit for obtaining image information
   c) emitting a shockwave with a shockwave source **characterised by**
   d) receiving reflected and/or scattered parts of the shockwave with the ultrasonic unit for obtaining image information of the shockwave focus.

16. Method according to claim 15, **characterised in that** the same transducer (2; 2'; 4; 4'; 5; 7) of the ultrasonic unit receives the reflected and/or scattered parts of the shockwave for further processing of the signals which also receives the reflections of the emitted ultrasound for further processing.

17. Method according to one of claims 15 or 16, **characterised in that** a transducer (2; 4) of the ultrasonic unit receives the reflected and/or scattered parts of the shockwave for further processing and a different transducer (2'; 4') of the ultrasonic unit receives the reflections of the emitted ultrasound for further processing.

18. Method according to one of claims 15 to 17, **characterised in that** the emission and reception of the ultrasound takes place at different times and/or at the same time as the reception of the reflected and/or scattered parts of a shockwave.

**Revendications**

1. Appareil de thérapie par ondes de choc avec :

   une source d'ondes de choc pour émettre une onde de choc, et

une unité d'ultrasons (1) pour la collecte d'informations d'image à l'aide de l'émission et de la réception d'ultrasons, étant précisé que

l'unité d'ultrasons est destinée à recevoir des parts réfléchies et/ou dispersées de l'onde de choc pour la collecte d'informations d'image,

**caractérisé en ce qu'**avec l'unité d'ultrasons une collecte d'informations d'image d'un foyer d'onde de choc est possible.

2. Appareil de thérapie par ondes de choc selon la revendication 1, **caractérisé en ce que** l'unité d'ultrasons comprend un ou plusieurs transducteurs (2 ; 2' ; 4 ; 4' ; 5 ; 7) qui comprennent chacun plusieurs éléments de transducteur (3a, 3b ; 3c ; 3a' ; 6a ; 6b ; 8a ; 8b) tels que des éléments piézoélectriques.

3. Appareil de thérapie par ondes de choc selon la revendication 2, **caractérisé en ce que** les éléments de transducteur électriques (3a, 3b ; 3c ; 3a' ; 6a ; 6b ; 8a ; 8b) sont disposés sur une ligne droite et/ou courbe telle qu'une ligne en arc de cercle, et/ou sur une surface plane et/ou courbe.

4. Appareil de thérapie par ondes de choc selon l'une des revendications 1 à 3, **caractérisé en ce que** l'unité d'ultrasons comprend des transducteurs (2 ; 2' ; 4 ; 4' ; 5 ; 7) avec différentes caractéristiques de fréquence et/ou **en ce qu'**un ou plusieurs transducteurs comprennent des éléments de transducteur (3a, 3b ; 3c' ; 3a' ; 6a ; 6b ; 8a ; 8b) avec différentes caractéristiques de fréquence.

5. Appareil de thérapie par ondes de choc selon l'une des revendications 1 à 4, **caractérisé en ce qu'**il est prévu un traitement électronique des signaux reçus par l'unité d'ultrasons, grâce auquel une focalisation de réception peut être réalisée.

6. Appareil de thérapie par ondes de choc selon l'une des revendications 1 à 5, **caractérisé en ce que** l'émission et la réception de l'ultrason peut se faire à des moments différents et/ou en même temps que la réception des parts réfléchies et/ou dispersées d'une onde de choc.

7. Appareil de thérapie par ondes de choc selon l'une des revendications 1 à 6, **caractérisé en ce que** l'information d'image collectée grâce à l'émission et à la réception et les informations d'image collectées grâce à la réception de parts réfléchies et/ou dispersées de l'onde de choc sont traitées en vue d'une représentation sur une seule image.

8. Appareil de thérapie par ondes de choc selon l'une des revendications 1 à 7, **caractérisé en ce que** la collecte d'informations d'image à l'aide de l'émission et de la réception d'ultrasons sert à produire une présentation B.

9. Appareil de thérapie par ondes de choc selon l'une des revendications 1 à 8, **caractérisé en ce qu'**il est prévu un capteur pour détecter le moment de l'émission d'une onde de choc.

10. Appareil de thérapie par ondes de choc selon l'une des revendications 1 à 9, **caractérisé en ce que** l'unité d'ultrasons est destinée à recevoir les ultrasons avec une fréquence située entre 1 MHz et 8 MHz.

11. Appareil de thérapie par ondes de choc selon l'une des revendications 1 à 10, **caractérisé en ce qu'**il est prévu des éléments de transducteur (3a ; 3b ; 3c ; 6a ; 6c ; 8a) qui présentent un maximum de sensibilité de réception situé au-dessous d'une fréquence spécifique, et **en ce qu'**il est prévu des seconds éléments de transducteur (3a' ; 3b' ; 3c' ; 6b ; 6d ; 8b) qui présentent un maximum de sensibilité de réception situé au-dessus de la fréquence spécifique, la fréquence spécifique étant par exemple de l'ordre de 0,5, 1, 1,5, 2, 2,5, 3, 3,5, 4, 4,5, 5, 5,5, 6, 6,5, 7, 7,5, 8, 8,5, 9, 9,5 ou 10 MHz ou entre ces valeurs.

12. Appareil de thérapie par ondes de choc selon l'une des revendications 1 à 11, **caractérisé en ce que** la source d'ondes de choc émet des ondes de choc avec un maximum d'intensité à une fréquence située au-dessous de 1 MHz.

13. Appareil de thérapie par ondes de choc selon l'une des revendications 1 à 12, **caractérisé en ce que** la source d'ondes de choc est une source d'ondes de choc électromagnétique.

14. Appareil de thérapie par ondes de choc selon l'une des revendications 1 à 13, **caractérisé en ce que** l'unité d'ultrasons est disposée de manière centrée devant la source d'ondes de choc.

15. Procédé pour collecter des informations d'image pour la thérapie par ondes de choc, avec les étapes qui consistent à :

a) émettre des ultrasons avec une unité d'ultrasons (1),

b) recevoir les réflexions de l'ultrason émis, avec l'unité d'ultrason en vue de la collecte d'informations d'image,

c) émettre une onde de choc avec une source d'ondes de choc,

**caractérisé par** l'étape qui consiste à

d) recevoir des parts réfléchies et/ou dispersées de l'onde de choc avec l'unité d'ultrasons en vue de la collecte d'informations d'image du foyer d'ondes de choc.

16. Procédé selon la revendication 15, **caractérisé en ce que** c'est le même transducteur (2 ; 2' ; 4 ; 4' ; 5 ; 7) de l'unité d'ultrasons qui reçoit les parts réfléchies et/ou dispersées de l'onde de choc pour le traitement des signaux et qui reçoit aussi les réflexions de l'ultrason reçu, en vue du traitement.

17. Procédé selon l'une des revendications 15 à 16, **caractérisé en ce qu'**un transducteur (2 ; 4) de l'unité d'ultrasons reçoit les parts réfléchies et/ou dispersées de l'onde de choc, en vue du traitement, et un autre transducteur (2' ; 4') de l'unité d'ultrasons reçoit les réflexions de l'ultrasons émis, en vue du traitement.

18. Procédé selon l'une des revendications 15 à 17, **caractérisé en ce que** l'émission et la réception de l'ultrason se fait à des moments différents et/ou en même temps que la réception des parts réfléchies et/ou dispersées d'une onde de choc.

FIG. 1

FIG. 2

$S_n$

X

Z

$\overrightarrow{r_n} - \overrightarrow{r_l}$

$\overrightarrow{r_l}$

$\overrightarrow{r_{l,z}}$

Phasenfront Stoßwelle

FIG. 3

X

Z

$\overrightarrow{r_n}$

$S_n$

$\overrightarrow{r_l}$

$\overrightarrow{r_{l,z}}$

$\overrightarrow{r_n} - \overrightarrow{r_l}$

Phasenfront Stoßwelle

FIG. 4

B-Bild

Abbildung des
Stroßwellenfokus

FIG. 5

FIG. 6a

FIG. 6b

FIG. 7a

FIG. 7b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 20315924 U1
- DE 3703335 C2
- EP 0460536 A1
- DE 4113697 A1
- WO 0013538 A
- US 5658239 A
- US 5769790 A
- DE 3328039
- DE 3119295 A1
- DE 3703338 A1
- DE 3617032